Europäisches Patentamt

European Patent Office

Office européen des brevets

(11) Publication number: **0 433 113 B1**

# EUROPEAN PATENT SPECIFICATION

(45) Date of publication of patent specification: **03.05.95**  (51) Int. Cl.[6]: **C07K  14/48**, A61K 38/00, A61K 38/27

(21) Application number: **90403221.6**

(22) Date of filing: **14.11.90**

(54) **Stabilization and maintenance of the nerve growth factor biological activity by use of natural gangliosides or derivatives thereof.**

(30) Priority: **14.11.89 IT 4855589**

(43) Date of publication of application:
**19.06.91 Bulletin  91/25**

(45) Publication of the grant of the patent:
**03.05.95 Bulletin  95/18**

(84) Designated Contracting States:
**AT BE CH DE DK ES FR GB GR IT LI LU NL SE**

(56) References cited:
**EP-A- 0 327 769**
**EP-A- 0 351 808**

**PROC. NATL. ACAD. SCI. USA, vol. 86, no. 6, March 1989, pp. 2056-2060, Washington, DC, US; A.C. CUELLO et al.**

**JOURNAL OF LIPID RESEARCH, vol. 25, no. 11, November 1984, pp. 1233-1245, New York, US; B. URLICH-BOTT et al.:**

(73) Proprietor: **FIDIA S.p.A.**
**Via Ponte della Fabbrica 3-A**
**I-35031 Abano Terme (Padova) (IT)**

(72) Inventor: **Della Valle, Francesco**
**Via Cerato 14**
**I-35100 Padova (IT)**
Inventor: **Romeo, Aurelio**
**Piazza Spagna 3**
**Rome (IT)**
Inventor: **Callegaro, Lanfranco**
**Via Bravi 35**
**I-35020 Ponte Di Brenta,**
**Padova (IT)**
Inventor: **Leon, Alberta**
**Piazza Napoli 19**
**Padova (IT)**

(74) Representative: **Hirsch, Marc-Roger et al**
**Cabinet Hirsch**
**34 rue de Bassano**
**F-75008 Paris (FR)**

**Description**

**BACKGROUND OF THE INVENTION**

**1. FIELD OF THE INVENTION**

The present invention concerns new, stable and biologically active complex compounds between the proteins and gangliosides, namely, stable and biologically active complexes between the $\beta$ subunit of Nerve Growth Factor ($\beta$NGF) and natural gangliosides or their semisynthetic analogues such as carboxy esters and amides or physiologically acceptable salts thereof; as well as pharmaceutical compositions containing such complexes, their therapeutic uses and a process for their manufacture.

**2. DESCRIPTION OF RELATED ART**

Gangliosides, i.e., glycosphingolipids containing sialic acid, are normally present in the cellular membranes of mammals and are found in their highest concentration, in nervous tissue. The location of gangliosides in the outer layer of the plasma membrane suggests that they play an important role in cell recognition, growth and differentiation. It is known that the composition of gangliosides and other conjugate glycolipids of the cell surface change during differentiation and during cell maturation processes. Four particular gangliosides, namely $GM_1$, GD1a, GD1b and GT1b, account for approximately 80-90% of all gangliosides in a normal human brain.

Gangliosides are comprised of a main, hydrophile, sialosyloligosaccharide group and a hydrophobic ceramide portion which consists of a sphingoside acylated at the nitrogen atom with fatty acids.

Gangliosides are effective in bringing about plastic changes in neurons; they enhance neurite growth in cell cultures and axonal growth in the central and peripheral nervous systems.

It is becoming increasingly evident that not only the development at the nervous system but also repair of the nervous system is controlled by extracellular signals which act on molecules at the cell surface.

Although detailed studies on the manifestation and function of glycolipids on the neuronal cell surface have only just begun, gangliosides already appear to play a prominent role in the regulation of both neuronal development and repair. During neuronal development, the characteristics of cerebral gangliosides undergo considerable quantitative and qualitative changes.

It has been reported that administration of gangliosides, in vivo, facilitates nerve regeneration and functional healing of the peripheral nervous system (PNS) after impairment (Ceccarelli B. et al., Adv. Exp. Med. Biol. 71:275, Plenum Press, New York, 1976; Gorio A. et al. Neuroscience 8:417, 1983). It has also been reported that the administration of gangliosides, particularly the monosialoganglioside known as $GM_1$, (nomenclature according to Svennerholm. J. Neurochemistry 10, 613, 1963), improves the consequences as a result of damage to the central nervous system (CNS) (Toffano G. et al., Brain Res. 261:163, 1983; Cuello A.C. et al., Dev. Brain Res. 376:373, 1986). Various recent studies indicate that gangliosides may have a modulatory function in the regulation of the in vitro activity of neuronotrophic factor. For example, it has been reported that the exogenous ganglioside $GM_1$ potentiates the growth of axons in PC-12 which is cells stimulated by the nerve growth factor (NGF), (Matta S.G. et al., Dev. Brain Res., 27:243, 1986) and similarly, $GM_1$ enhanceneuritic growth induced by NGF, of gangliar neurons of dorsal embryonic origin and sympathetic ganglia induced by NGF (Skaper S.D. et al., Int. J. Dev. Neurosci. 3:187, 1985; Skaper S.D. et al., Dev. Brain Res. 23:19, 1985).

The results of recent studies to assess the possibility that $GM_1$ in vivo influences NGF activity suggest that the neuronotrophic factor NGF presumably plays a primary role in the maintenance of cell survival following disease in the central nervous system. (Cuello A.C. et al., in: A new intramembrane integrative mechanism 62, Kjell Kuxe and L. Agnati Editors, 1987) Interestingly, it has been reported that treatment with $GM_1$ facilitates neuronal cell survival, not only in dopaminergic and noradrenergic neurons, but also in cholinergic neurons known to respond to NGF.

Mixtures of $GM_1$ and $\beta$-NGF wherin $GM_1$ is capable of binding $\beta$-NGF with low affinity are described in Proc.Natl.Acad.Sci., 1989, 86, pp. 2056-2060.

The indispensability of NGF for the survival of sympathetic neurons and target innervation during development constitutes the chief evidence of the essential, trophic role of this growth factor on its target cells.

Considerable correlation has been observed between levels of NGF and the distribution of magnocellular cholinergic neurons. Relatively high levels of NGF, in the range of those observed in a known target, the peripheral sympathetic tissue, have been observed both in the innervated regions of magnocellular cholinergic neurons and in the regions containing their cellular bodies, such as the hippocampus. This relationship between the integrity of the basal proencephalic cholinergic system and cognitive functions could also be present in man. One of the main neuropathological characteristics of Alzheimer's disease is a drastic loss of magnocellular cholinergic neurons, although other transmitter systems also undergo various forms of mutation. Therefore, possible links between NGF and the physiopathology and potential therapy for Alzheimer's disease should now be seriously considered.

The possibility of producing human NGF by recombinant DNA biotechnological methods is the basis of an experimental investigation. Regarding the therapeutic outcome, the benefits observed after experimental lesion of the cholinergic system suggest that there could be a substantial increase in the availability of NGF for these neurons, both by exogenous application and by stimulation of endogenous production. The development of biotechnological methods has produced ever increasing numbers of proteins and their peptides for potential therapeutic use. These macromolecules present particular and complex problems regarding drug administration. For the time being, the only way to systemically administer these pharmacologically active peptides is by subcutaneous, intramuscular or intracranial injection, especially for NGF. Treatment regimens requiring several injections per day or week have various disadvantages, including patient noncompliance and unfavorable pharmacokinetics, in particular for protein-substituting therapies.

One of the aims of research in this field is to develop chemical methods to increase the stability of proteins without impeding their biological activity. Various biological products have been developed through liposome technology. However, these biological products present various problems, such as in the poor specificity of the chemical substances used to trap the proteins, their a specific interaction in vivo with organs, poor reproducibility from batch to batch of the complex compound, and the unknown pharmacokinetics of the chemical compounds used to trap the proteins.

By assessing the problems and potential pharmaceutical application of the neuronotrophic factors on the one hand and the biological activity of the gangliosides in vivo on the other, it has been possible to prepare a stable complex of the two types of compounds which is biologically active, and satisfies the aforesaid requisites of specificity, stability and selective bioavailability. These qualities can be demonstrated in experimental models both in vitro and in vivo.

## SUMMARY OF THE INVENTION

The present invention, therefore, concerns mainly complexes formed between the $\beta$ subunit of the neuronotrophic factor NGF and a natural ganglioside or a semisynthetic ganglioside analogue, which is obtained by contact of the two components dissolved in an aqueous solution at room temperature under slightly alkaline conditions.

## BRIEF DESCRIPTION OF THE DRAWINGS

FIG. 1 shows an immunoblot with $GM_1$-NGF ($\beta$ subunit) complex after reaction with affinity purified anti-mouse NGF ($\beta$ subunit) immunoglobulins.

FIG. 2 shows the effects of the $GM_1$-$\beta$NGF complex in presenting the VNB-induced reduction in NA in mouse heart on the 6th day of life.

## DETAILED DESCRIPTION OF THE PREFERRED EMBODIMENTS

The ratio in weight between the two components of the complex can vary greatly, but is preferably between 1:100,000 and 1:10 of neuronotrophic factor and ganglioside, respectively. Within these limits, the most important compounds are those with a neuronotrophic factor:ganglioside ratio of 1:1000.

The Nerve Growth Factor $\beta$ subunit ($\beta$NGF) to be used according to the present invention can be isolated from human or animal tissue by known methods; for example, from mammals, or from cell culture mediums, should human $\beta$NGF obtained by recombinant DNA techniques be used: it is preferable to use preparations obtained from human placenta tissue or from bovine seminal fluid.

$\beta$NGF can, however, also be prepared by molecular biology techniques; for example, by recombinant DNA technology as disclosed by EP-B-121,338.

The new complexes according to the present invention have the same type of biological activity as $\beta$NGF, as can be demonstrated, for example, by experiments in vitro on a PC-12 cell line. They can therefore be used therapeutically in all cases where $\beta$NGF-based preparations are indicated; for example, in the aging of the nervous system. The new complexes can also be used in therapy or in preventive medicine where the particular action of gangliosides, which is maintained in the new complexes, is useful; for example, immunochemical activity, as can be seen in the action of the complex compound of $\beta$NGF with the ganglioside $GM_1$ against anti-$GM_1$ polyclonal antibodies.

The biological activity of the new $\beta$NGF-ganglioside complexes and their derivatives can be demonstrated on laboratory animals as described in example 1 for the case of the complex compound $\beta$NGF-$GM_1$.

Examples of semisynthetic ganglioside analogues, which are useful, are functional derivatives, such as esters or amides of the sialic carboxy groups, described for example in U.S. Patent No. 4,713,374; inner esters, such as those described in U.S. Patent No. 4,593,091 and in European Patent No. 0072 722. Derivatives which are peracylated on the sialic, saccharide and possibly ceramide hydroxy groups described in U.S. Patent No. 4,713,374 are also functional derivatives. Other examples of semisynthetic ganglioside analogues which are useful, are those called lysogangliosides; that is, gangliosides deacylated on the sphingosine nitrogen and/or de-N-acetyl-gangliosides and de-N-acetyl-lysogangliosides wherein the nitrogen of the neuraminic residue is also deacylated; as well as derivatives analogous to gangliosides which have superior fatty acids, such as palmitic or stearic acid, or acyl groups derived from any other acid of the aliphatic, aromatic or alicyclic or heterocyclic series, possibly also substituted by other functional groups, especially by polar groups. In other semisynthetic analogues, apart from the "unnatural" acyl groups mentioned here, acyl groups which differ from the acetyl group on the neuraminic nitrogen may also be present.

Semisynthetic compounds and their functional derivatives, such as esters, amides, inner esters and peracylate derivatives useful in the complex of the present invention are known and/or described un U.S. Patent No. 4,593,091 and in U.S. Patent No. 4,713,374.

Any ganglioside which has a NANA group, i.e., N-acetyl-neuraminic acid, can form a complex with $\beta$NGF according to the present invention.

By the term complex is meant an association between the NANA group of the ganglioside with the $\beta$ subunit of NGF. This association is the result of ionic and hydrogen interaction between the two compounds which distinguish the complex of the present invention from a mere mixture of ganglioside and $\beta$NGF.

Gangliosides serving as a basis for the preparation of the new complexes are preferably those chosen from the group formed by the aforesaid gangliosides constituting the greater part of brain gangliosides, that is, $GM_1$, GD1a, GD1b and DT1b and aforesaid functional derivatives and semisynthetic analogues derived therefrom.

According to the present invention, ganglioside salts can also be used for the above said purposes; for example, metal salts, such as salts of alkaline and alkaline earth metals or aluminium and magnesium or salts with organic bases, preferably therapeutically acceptable nitrogenous. bases, as, for example, salts of aminoalcohols such as ethanolamine.

Furthermore, according to the present invention, it is possible to obtain complexes between the neuronotrophic factor and acid addition salts of gangliosides or their above mentioned derivatives; for example, hydrochlorides, sulfates, nitrates, mesylates, and tartrates.

All of the aforesaid properties and uses relating to the new complexes between the $\beta$NGF factor and gangliosides or their derivatives can be attributed also to the aforesaid salts, which are, therefore, a particular aspect of the present invention.

Special emphasis should be placed on those complexes derived from the following esters and amides of the ganglioside $GM_1$; the aliphatic esters derived from alcohols with a maximum of 8 carbon atoms, such as methyl, ethyl, propyl, isopropyl alcohol, butyl, amyl alcohols and hexyl alcohols, such as n-hexyl alcohol and n-octyl alcohol, and the aliphatic amides derived from amines with a maximum of 8 carbon atoms, such as methylamine, ethylamine, propylamine, hexylamine or octylamine.

Of the derivatives peracylated at the ganglioside hydroxyls groups, include but are not limited to acetates, propionates, butyrates, valerianates, succinates and malonates.

The preparation procedure of the new complexes between the aforesaid proteins and gangliosides or their derivatives or their salts consists essentially of bringing the two components of the resultant complex into contact with each other in an aqueous solution, in the presence or absence of other organic solvents, such as inferior aliphatic alcohols, or lower ketones or lower dialkylamines or sulfoxides; that is, at room temperature or somewhat higher; for example, 50 °C, and in a slightly alkaline medium; for example, in the presence of inorganic or organic bases, such as ammonia, or inferior aliphatic amines, or alkaline or alkaline earth hydrates or the corresponding carbonates or acetates. Under these conditions ganglioside salts are clearly formed which, being soluble in water, facilitate the condensation reaction. The neuronotrophic factor, too, can be dissolved in an aqueous solvent, such as an alkaline acetate, or it can also be suspended in the ganglioside solution.

Reaction can last for anything from a few hours to several days, but normally runs between 12 and 24 hrs.

The protein-ganglioside derivative complex can be isolated from the reaction mixture by known methods, such as those illustrated in Example 1, and can by identified, due to its biological activity and immunochemical recognition with monoclonal and polyclonal antibodies specific to the protein, or sedimentation systems using ultracentrifuge.

An further object of the present invention is also a pharmaceutical composition containing as the active substances one or more of the new complexes between the $\beta$ subunit of NGF and a ganglioside one of its aforesaid derivatives or semisynthetic analogues or their salts, and in particular those discussed in detail above. Such pharmaceutical composition can be for oral, rectal, parenteral, or local uses. They can therefore be in solid form or semisolid form; for example, pills, tablets, gelatinous capsules, capsules, suppositories, or soft gelatin capsules. For parenteral use, it is possible to use those forms useful for intramuscular or subcutaneous administration, for infusions, for intravenous or for intracerebral injection. As a result, they can therefore be presented as solutions of the active compounds, or as freeze-dried powders of the active compounds to be mixed with one or more pharmaceutically acceptable excipients or diluents which are suitable for the above uses and which have an osmolarity compatible with the physiological fluids.

For local use, preparations in the form of sprays; for example, inhalation sprays, creams, or ointments for topical use are useful.

The preparations of the invention can be administered to humans or animals. They contain preferably between 0.01% and 10% of active ingredient in the case of solutions, sprays, ointments and creams and between 1% and 100% and preferably between 5% and 50% of active compound in the case of solid preparations. The dosage to be administered depends on the individuals requirements, on the desired effect and on the chosen route of administration.

The invention also includes the therapeutic use of all the complexes of gangliosides or their derivatives with the $\beta$ subunit of the neuronotrophic growth factor NGF for the aforesaid indictions.

Daily doses to man vary between 0.01 to 100 mg/kg body weight.

Daily dosages to man by injection (subcutaneous or intramuscular or intracerebral) vary between 0.05 mg and 5 mg of active substance per kg of body weight.

The following examples describe how the new compounds are prepared, the procedure for their preparation, the pharmaceutical preparations and their uses.

## EXAMPLE 1

### MATERIALS AND METHODS

Mouse NGF ($\beta$ subunit) was purified according to the method described by Angeletti P.V. et al., Natl. Acad. Sci. USA 64, 787, 1969. in vitro studies were carried out using "dissociated" fetal cells from E8 chick dorsal root ganglia (DRG E-8), as described by Skaper S.D. et al., Exp. Neurol. 76:655, 1982. The study of NGF antibodies raised against mouse NGF to inhibit the biological activity of NGF ($\beta$ subunit), was assessed using the aforesaid in vitro model.

The immunoreactivity of $GM_1$, NGF ($\beta$ subunit) and the $GM_1$-NGF ($\beta$ subunit) complex was measured by the immunoblot technique.

NGF polyclonal antibodies against mouse NGF ($\beta$ subunit) were purified by affinity chromatography using 2.5S mouse NGF bound to Sepharose 4B as described in K. Stoeckel et al., J. Neurochem. 26:1207, 1976.

$GM_1$ polyclonal antibodies against monosialoganglioside $GM_1$ are purified by affinity chromatography with $GM_1$ immobilized Sepharose 4B.

Isopycnic sedimentation of NGF ($\beta$ subunit) and of the ganglioside complexes was conducted as described by Ulrich-Bott B. et al., Journal of Lipid Research 25, 1233, 1984.

Monosialoganglioside $GM_1$ (for example 50 mg) was dissolved in $CHCl_3$/MeOH (ratio 2:1). The solvent was then evaporated under nitrogen. Monosialoganglioside $GM_1$ was resuspended in 50 mM NH4OH, pH 8.5 for one hr. The concentration of monosialoganglioside $GM_1$, for example at 10 mg/ml, and highly purified mouse NGF ($\beta$ subunit), was dissolved in 50 mM of sodium acetate, pH 5.0, and incubated to obtain a $\beta$NGF-$GM_1$ weight ratio of 1:1000. The mixture was incubated at pH 8.5 under stirring; for example, overnight at room temperature. The same results were obtained at 4 °C.

The biological complex $\beta$NGF-$GM_1$ was purified from unreacted $\beta$NGF by anionic exchange chromatography; for example, on an analytical mono Q column, and equilibrated with 50 mM NH4OH, pH 8,5. The $\beta$NGF-$GM_1$ complex was eluted using ammonium acetate with a 0-1.0 M gradient in 50mM NH4OH, pH 8.5.

The flow rate was 1ml/min. The unreacted $\beta$NGF was eluted with the void volume. The $\beta$NGF-GM$_1$ complex was eluted in the time range of 15-18 minutes with an ammonium acetate concentration of 0.15-0.25 M. These were the experimental conditions under which 12 micrograms of NGF ($\beta$ subunit) were bound to 1 mg of monosialoganglioside GM$_1$.

The chemical, immunochemical and biological characteristics of the $\beta$NGF-GM$_1$ complex were assessed in vitro as follows:

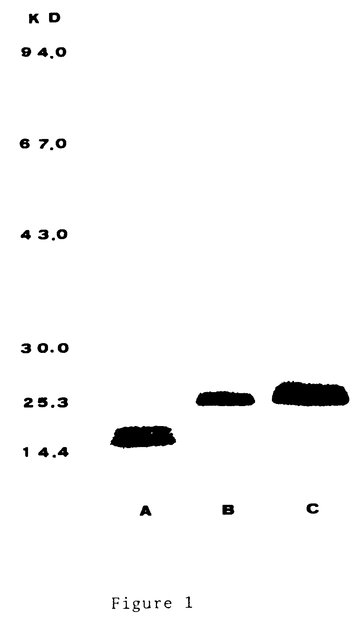

a) The purified $\beta$NGF-GM$_1$ complex showed in the immunoblot technique maintenance of its immunoreactivity against purified polyclonal antibodies raised against mouse NGF ($\beta$ subunit) (Fig. 1).

b) The purified $\beta$NGF-GM$_1$ complex showed biological activity on DRG-E8.

c) The biological activity of the $\beta$NGF-GM$_1$ complex was inhibited by affinity purified polyclonal antibodies at a concentration of 60 $\mu$g/ml and by the monoclonal antibody at a concentration of 500 $\mu$g/ml. The biological activity of $\beta$NGF without GM$_1$ was inhibited by affinity purified polyclonal antibodies at a concentration of 30 $\mu$g/ml and by monoclonal antibody at a concentration of 100 $\mu$g/ml.

d) By the immunoblot technique, the same band, correlated with the biologically active $\beta$NGF-GM$_1$ complex, showed reactivity against polyclonal antibodies, raised against NGF ($\beta$ subunit) and raised against monosialoganglioside GM$_1$.

e) The biological activity of $\beta$NGF-GM$_1$, assessed on DRG-E8, was maintained after incubation of the $\beta$NGF-GM$_1$ both in a low-pH buffer and in the presence of bivalent ions (for example, 40 mM CaCl2).

f) Stability of the $\beta$NGF-GM$_1$ complex was maintained in the presence of human serum, at 37°C for 96 hrs.

g) The biological activity of the $\beta$NGF-GM$_1$ complex was maintained after digestion with proteolytic enzyme (for example, pepsin at 2%, w/w, for 16 hrs, pH 2.0 at room temperature). Under the same conditions the proteolytic enzyme inhibits the activity of $\beta$NGF without GM$_1$.

h) The biological activity of the $\beta$NGF-GM$_1$ complex was maintained after its storage at different temperatures (for example, 4°C, 37°C, 45°C). Under the same storage conditions, in terms of time and protein concentration, the $\beta$NGF without monosialoganglioside GM$_1$, completely lost its biological activity.

i) The $\beta$NGF-GM$_1$ complex, assessed by isopycnic sedimentation, settles at a density of 1.298 g/cm3.

Immunoblot with GM$_1$-NGF ($\beta$ subunit) complex after reaction with affinity purified anti-mouse NGF ($\beta$ subunit) immunoglobulins is shown in Figure 1.

A run: GM$_1$-NGF complex before chromatography.

B run: asialo GM$_1$-$\beta$NGF complex.

C run: mouse NGF, $\beta$ subunit, in its dimeric form, GM$_1$ and $\beta$NGF were incubated as previously described for 2 hrs at room temperature. The incubation of asialo GM$_1$ and NGF was effected under the same conditions.

The biological activity in vivo of the $\beta$NGF-GM$_1$ complex was assessed in animal models, in which the biological activity of $\beta$NGF had been suppressed by a chemical compound. New-born Sprague-Dawley mice of both sexes were used. All systemic injections were administered subcutaneously with 0.01 ml/g of body weight. Solutions of vinblastine (VNB) sulfate were prepared with saline and a dose of 0.15 mg/kg was administered to each mouse on the third day of its life. The mice treated with VNB were subdivided into three groups: group 1 received saline solution, group 2 $\beta$NGF (for example, 0.1 mg/kg of $\beta$NGF), group 3 $\beta$NGF-GM$_1$ complex (for example, 0.1 mg/kg of $\beta$NGF and 30 mg/kg of monosialoganglioside GM$_1$). The animals were sacrificed on the sixth day of life. Their hearts were rapidly removed, frozen in dry ice and stored at -80°C until ready for use.

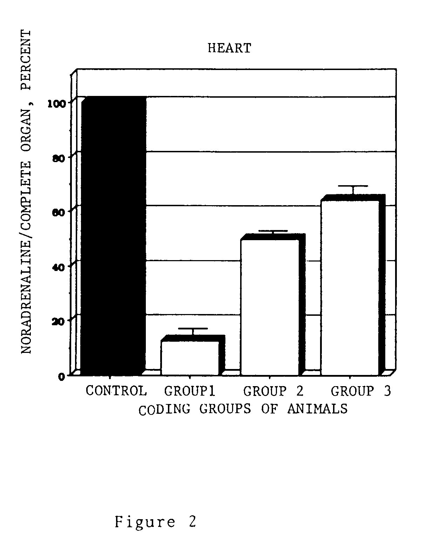

The quantity of noradrenaline (NA) in the heart was assessed. As reported in Fig. 2, VNB administration causes a considerable loss of NA content in the heart (group 1), a significantly antagonizing effect with the administration of both $\beta$NGF without GM$_1$ (group 2) and $\beta$NGF-GM$_1$ complex.

Moreover, these results indicate that the $\beta$NGF-GM$_1$ complex is biologically efficacious on the sympathetic nervous system in vivo and is more biologically active than $\beta$NGF.

EXAMPLE 2

The NGF-ganglioside complex consists of monosialoganglioside GM$_1$ and human NGF ($\beta$ subunit) obtained by recombinant DNA technology, exactly as described in example n. 1. Its properties in vitro and in vivo are those reported in Example 1.

EXAMPLE 3

The complex between ganglioside GD1a and $\beta$NGF, purified, for example, from submaxillary glands is prepared under the experimental conditions reported in Example 1.

GD1a-$\beta$NGF maintains the biological activity assessed on DRG-E8. This complex, assessed by isopycnic sedimentation, settles at a density of 1.3496 g/cm3.

EXAMPLE 4

The complex between ganglioside GD1b and $\beta$NGF, purified, for example, from submaxillary glands is prepared under the experimental conditions reported in Example 1.

GD1b-$\beta$NGF maintains the biological activity assessed on DRG-E8. This complex, assessed by isopycnic sedimentation, settles at a density of 1.3596 g/cm3.

EXAMPLE 5

The complex between GT1b and $\beta$NGF, purified, for example, from submaxillary glands, is obtained under the experimental conditions reported in Example 1.

GT1b-$\beta$NGF maintains the biological activity assessed on DRG-E8. This complex, assessed by isopycnic sedimentation, settles at a density of 1.3700 g/cm3.

EXAMPLE 6

The complex between the inner ester of $GM_1$ and NGF $\beta$ subunit is prepared under the experimental conditions reported in Example 1.

EXAMPLE 7

The complex between the methyl ester of $GM_1$ and NGF $\beta$ subunit is prepared under the experimental conditions reported in Example 1.

EXAMPLE 8

The complex between the ethyl ester of $GM_1$ and NGF $\beta$ subunit is prepared under the experimental conditions reported in Example 1.

EXAMPLE 9

The complex between the isopropyl ester of $GM_1$ and NGF $\beta$ subunit is prepared under the experimental conditions reported in Example 1.

EXAMPLE 10

The complex between the tert-butyl ester of $GM_1$ and NGF $\beta$ subunit is prepared under the experimental conditions reported in Example 1.

EXAMPLE 11

The complex between the benzyl ester of $GM_1$ and NGF $\beta$ subunit is prepared under the experimental conditions reported in Example 1.

EXAMPLE 12

The complex between the methylamide derivative of $GM_1$ and NGF $\beta$ subunit is obtained under the experimental conditions reported in Example 1.

EXAMPLE 13

The complex between the ethylamide derivative of $GM_1$ and NGF $\beta$ subunit is obtained under the experimental conditions described in Example 1.

EXAMPLE 14

The complex between the benzylamide derivative of $GM_1$ and NGF $\beta$ subunit is obtained under the experimental conditions reported in Example 1.

EXAMPLE 15

The complex between the isopropylamide derivative of $GM_1$ and NGF $\beta$ subunit is obtained under the experimental conditions reported in Example 1.

All the complexes obtained between the various ganglioside derivatives and nerve growth factor $\beta$ subunit presented biological activity when assessed on DRG-E8 cultures.

PHARMACEUTICAL PREPARATIONS

The formulation of pharmaceutical preparations containing ganglioside-$\beta$NGF complex includes known methods for the preparation of pharmaceutically acceptable compositions, for administration to patients, and in which it is possible for an effective quantity of the complex to be combined in a mixture with a pharmaceutically acceptable vehicle.

Suitable vehicles and their formulations inclusive of other proteins, are described, for example, in the book "Remington's Pharmaceutical Sciences" (Remingtons's Pharmaceutical Sciences, Mack Publishing Company, Easton, Pa., USA 1985). These vehicles include injectable "deposit formulations".

On this basis, the pharmaceutical formulation contains, albeit not exclusively, solutions of $GM_1$-$\beta$NGF complex or freeze-dried powders of $GM_1$-$\beta$NGF complex in association with one or more pharmaceutically acceptable vehicles or diluents, and contained in buffered media at a suitable pH, and isosmotic with the physiological fluids. The various formulations of $GM_1$-$\beta$NGF complex can be considered examples of preparations for pharmaceutical use also for ganglioside derivative-$\beta$NGF complexes, formulations which depend on the desired biological activity and route of administration. Table 1 shows, for illustrative purposes only and without limiting the same, examples of the formulations which can be prepared in the form of solutions for the treatment of dysfunctions of the nervous system. In the case of freeze-dried preparations, supporting excipients such as, but not exclusively, mannitol or glycine may be used and appropriate buffered solutions of the desired volume will be provided so as to obtain adequate isotonic buffered solutions of the desired pH. Similar solutions may be used for the pharmaceutical preparations of the $GM_1$-$\beta$NGF complex in isotonic solutions of the desired volume and include, albeit not exclusively, the use of buffered saline solutions with phosphate or citrate at suitable concentrations so as to obtain at all times isotonic pharmaceutical preparations of the desired pH, for example, neutral pH.

For illustrative purposes, Tables 2 and 3 show examples of pharmaceutical preparations for the treatment of nervous system dysfunctions.

The pharmaceutical preparations set out in Table 3, System 4 and System 5, are prepared in twin vials for each single dose. The first vial contains the active substance with a composition in weight of approximately 0.01% to 50% of active substance together with a pharmacologically acceptable excipient, such as glycine or mannitol. The second vial contains a solvent prepared with the desired volume of buffered saline solution with phosphate or citrate. The contents of the two vials are mixed immediately before use and the freeze-dried active substance rapidly dissolves giving an injectable solution. Table 3 also shows a possible example of a pharmaceutical preparation for subcutaneous injection (System No. 5).

The complexes of the present invention may be made into pharmaceutical compositions by combination with appropriate medical carriers or diluents, and may be formulated into preparations in solid, semisolid, liquid or gaseous form such as tablets, capsules, powders, granules, ointments, solutions, suppositories, injections, inhalants, aerosols in usual ways for oral or parenteral administration. The following methods and excipients are merely exemplary and are in no way limiting.

In the case of inhalants or aerosol preparations, the compounds of the invention in the form of a liquid or minute powder may be filled up in an aerosol container with gas or liquid spraying agents, and if desired, with conventional adjuvants such as humidifying agents. They may also be applied as pharmaceuticals for a non-pressurized preparation such as in a nebulizer or an atomizer.

The pharmaceutical formulation also includes, but is not limited to, suppositories for rectal administration with lypophilic excipients, for example, hydrosoluble, autoemulsive excipients, such as glycogelatine or similar substances. In these preparations, the $GM_1$-$\beta$NGF complex may be present in quantities varying between 0.001% and 1% by weight of the total excipient. The suppositories may also further contain, suitable quantities of acetylsalicylate.

Table 4 lists, for illustrative purposes only, preparations in the form of suppositories for the treatment of nervous system dysfunctions.

The dosage of the pharmaceutical preparations of the $GM_1$-$\beta$NGF complex and the administration times depend on the desired effect (determined in clinical trials) and on the route of administration for example, the dosages and administration times may be similar (but not exclusively) to those normally used in studies with other neuronotrophic agents.

## TABLE 1

### Examples of pharmaceutical compositions for injectable solutions.

PREPARATION No. 1 - one 2 ml ampoule contains:

| | | |
|---|---|---|
| $GM_1$-ßNGF complex | 2 | mg Monosialoganglioside $GM_1$ |
| | 1 | µg (8.000 BU) ßNGF |
| Sodium chloride | 16 | mg |
| Citrate buffer pH 7 | | |
| in distilled water to | 2 | ml |

PREPARATION No. 2 - one 2 ml ampoule contains:

| $GM_1$-βNGF complex | 5 mg Monosialogan-glioside $GM_1$ |
| | 10 µg (8.000 BU) βNGF |
| Sodium chloride | 16 mg |
| Citrate buffer pH 7 | |
| in distilled water to | 2 ml |

The biological unit (BU) is defined in the article by Fenton, E.L., Expl. Cell Res. 59:383, 1970.

TABLE 2

Examples of pharmaceutical composition systems.

SYSTEM No. 1

a) one 2 ml vial contains:

| Freeze-dried active substance | 1.5 mg Monosialogan-glioside $GM_1$ |
| | 4 µg (32.000 BU) βNGF |
| Glycine | 30 mg |

b) one 2 ml vial of solvent contains:

| Sodium chloride | 16 mg |
| Citrate buffer in distilled water to | 2 ml |

SYSTEM No. 2

a) one 2 ml ampoule contains:

Freeze-dried

| | |
|---|---|
| active substance | 1.5 mg Monosialogan- |
| | glioside $GM_1$ |
| | 4 µg (32.000 BU)  ßNGF |
| Mannitol | 40 mg |

b) one 2 ml vial of solvent contains:

| | |
|---|---|
| Sodium chloride | 16 mg |
| Citrate buffer in | |
| distilled water to | 2  ml |

SYSTEM No. 3

a) one 3 ml ampoule contains:

Freeze-dried

| | |
|---|---|
| active substance | 3  mg Monosialogan- |
| | glioside $GM_1$ |
| | 10 µg (80.000 BU) ßNGF |
| Glycine | 45 mg |

b) one 3 ml vial of solvent contains:

| | |
|---|---|
| Sodium chloride | 24 mg |
| Citrate buffer in | |
| distilled water to | 3  ml |

TABLE 3

Example of pharmaceutical composition systems.

SYSTEM No. 4

a) one 3 ml vial contains:

Freeze-dried

active substance                    3   mg Monosialogan-

glioside GM$_1$

10 μg (80.000 BU) βNGF

Mannitol                           60 mg

b) one 3 ml vial of solvent contains:

Sodium chloride              24 mg

Citrate buffer in

distilled water to           3   ml

SYSTEM No. 5 (Example for subcutaneous injection)

a) one 2 ml ampoule contains:

Freeze-dried

active substance            1.5 mg. Monosialogan-

glioside GM$_1$

5   μg (40.000 BU) βNGF

Glycine                     30 mg

b) one 2 ml vial of solvent contains:

Sodium chloride             16 mg

Citrate buffer in

distilled water to       2  ml

TABLE 4

Examples of pharmaceutical compositions in the form of suppositories for rectal use.

PREPARATION No. 1

| GM$_1$-ßNGF complex | 3 mg Monosialogan- |
|---|---|
| | glioside GM$_1$ |
| | 10 µg (80.000 BU) ßNGF |
| Cocoa butter | 2.5 gr |

PREPARATION No. 2

| GM$_1$-ßNGF complex | 3 mg Monosialogan- |
|---|---|
| | glioside GM$_1$ |
| | 10 µg (80.000 BU) ßNGF |
| Carbowax 1540 | 1.75 gr |
| Carbowax 6000 | 0.75 gr |

PREPARATION No. 3

| GM$_1$-ßNGF complex | 3 mg Monosialogan- |
|---|---|
| | glioside GM$_1$ |
| | 10 µg (80.000 BU) ßNGF |
| Tween 61 | 2.125 gr |
| Lanolin | 0.25 gr |

PREPARATION No. 4

| | |
|---|---|
| GM$_1$-ßNGF complex | 3 mg Monosialogan-<br>glioside GM$_1$ |
| | 10 µg (80.000 BU)ßNGF |
| Glycerine | 1.5 gr |
| Water | 0.75 gr |
| Gelatine | 0.25 gr |

The invention being thus described, it is clear that these methods can be modified in various ways. Such modifications are not to be considered to depart from the spirit and scope of the invention and any modifications which might appear evident to an expert in the art come within the range of the invention.

**Claims**

1. A complex which consists of the $\beta$ subunit of NGF and a ganglioside, associated through its NANA group, selected from the group consisting of a natural ganglioside, a semisynthetic analogue of said natural ganglioside and a physiologically acceptable salt thereof in substantially pure form.

2. The complex according to claim 1, wherein the weight ratio between said $\beta$NGF factor and said ganglioside is from 1:100,000 to 1:10.

3. The complex according to claim 2, wherein the weight ratio between said $\beta$NGF and said ganglioside is approximately 1:1000.

4. The complex according to any one of claims 1-3, wherein said ganglioside is a natural ganglioside selected from the group consisting of GM1, GD1a, GD1b and GT1b.

5. The complex according to any one of claims 1-3, wherein said ganglioside is a lysoganglioside.

6. The complex according to any one of claims 1-3, wherein said ganglioside is a de-N-acetyl-ganglioside or a de-N-acetyl-lysoganglioside.

7. The complex according to any one of claims 1-3, wherein said ganglioside has a sphingosine acyl group and wherein said acyl group is derived from an acid other than those present in natural gangliosides and selected from the group consisting of an aliphatic, an aromatic, an alicyclic and a heterocyclic group and which can be optionally substituted by at least one functional group.

8. A complex according to one of claims 1-3, wherein said ganglioside is a ganglioside derivative having an acyl group, said acyl group: being, derived from an acid other than those present in natural gangliosides and selected from the group consisting of an aliphatic, an aromatic, an alicyclic and a heterocyclic group which can be optionally substituted by at least one functional group.

9. The complex according to any one of claims 1-3, wherein said ganglioside is a ganglioside derivative having both a sphingosine and a neuraminic amide group and wherein one or both of said groups are acylated by an acid other than those present in natural gangliosides and wherein said acid is selected from the group consisting of an aliphatic radical, an aromatic radical, an alicyclic radical and a heterocyclic radical and which can be optionally substituted by at least one functional group.

10. The complex according to any one of claims 1-3, wherein said semisynthetic analogue of a ganglioside is selected from the group consisting of semisynthetic analogues of GM1, GD1a, GD1b and GT1b.

14

**11.** The complex according to claim 1, wherein said ganglioside is an aliphatic ester of the ganglioside GM1, said aliphatic ester having a maximum of 8 carbon atoms.

**12.** The complex according to claim 1, wherein said ganglioside is an aliphatic amide of GM1, said aliphatic amide having a maximum of 8 carbon atoms.

**13.** The complex according to claim 1, wherein said ganglioside is a peracetylate, a perpropionylate, a perbutyrate, a pervalerianylate, a persuccinylate or a permalonylate of the ganglioside GM1.

**14.** The complex according to any one of claims 1-13 which is $\beta$NGF-GM1 complex.

**15.** The complex according to any one of claims 1-13 which is $\beta$NGF-GD1a complex.

**16.** The complex according to any one of claims 1-13 which is $\beta$NGF-GD1b complex.

**17.** The complex according to any one of claims 1-13 which is $\beta$NGF-GT1b complex.

**18.** The complex according to any one of claims 1-13 which is $\beta$NGF-GM1 inner ester complex.

**19.** A process for the preparation of a complex as claimed in claim 1, which comprises contacting the $\beta$ subunit of NGF with a ganglioside in an aqueous solution in the presence or absence of other organic solvents, at room temperature or slightly higher, under alkaline conditions; and collecting said complex.

**20.** The process according to claim 19, wherein said organic solvent is a lower dialkylamide or diethylsulfoxide.

**21.** The complex between the $\beta$ subunit of the NGF neuronotrophic factor and a natural ganglioside or a semisynthetic analogue of a ganglioside according to one of claims 1-18 in view of is therapeutic use.

**22.** The complex according to claim 21 for the treatment of neuropathological conditions.

**23.** The complex according to claim 21 or 22 for the treatment of the aging of the nervous system.

**24.** The complex according to claim 21 or 22 for the treatment of chronic neurodegenerative disease.

**25.** The complex according to claim 21 or 22 for the treatment of chronic immunological disease afflicting the nervous system.

**26.** A pharmaceutical composition which comprises an effective amount of a complex between the $\beta$ subunit of NGF and a natural ganglioside associated through its NANA group or a semisynthetic analogue of a ganglioside or a physiologically acceptable salt thereof; and a pharmaceutically acceptable carrier.

**27.** The use of a complex between the $\beta$ subunit of the neuronotrophic growth factor NGF and a ganglioside according to claim 1 for the preparation of a pharmaceutical composition for the treatment of neuropathological conditions.

**Patentansprüche**

**1.** Komplex bestehend aus der $\beta$-Untereinheit von NGF und einem durch seine NANA-Gruppe assoziierten Gangliosid, ausgewählt aus der Gruppe bestehend aus einem natürlichen Gangliosid oder halbsynthetischen Analogen des natürlichen Gangliosids oder einem physiologisch akzeptablen Salz davon in im wesentlichen reiner Form.

**2.** Komplex nach Anspruch 1, worin das Gewichtsverhältnis zwischen dem $\beta$NGF-Faktor und dem Gangliosid von 1 : 100000 bis 1 : 10 beträgt.

3. Komplex nach Anspruch 2, worin das Gewichtsverhältnis zwischen dem βNGF und dem Gangliosid ungefähr bei 1 : 1000 liegt.

4. Komplex nach einem der Ansprüche 1- 3, worin das Gangliosid ein natürliches Gangliosid ist ausgewählt aus der Gruppe bestehend aus GM1, GD1a, GD1b und GT1b.

5. Komplex nach einem der Ansprüche 1-3, worin das Gangliosid ein Lysogangliosid ist.

6. Komplex nach einem der Ansprüche 1-3, worin das Gangliosid ein De-N-acetylgangliosid oder ein De-N-acetyllysogangliosid ist.

7. Komplex nach einem der Ansprüche 1-3, worin das Gangliosid eine Sphingosinacylgruppe besitzt und worin die Acylgruppe von einer anderen Säure abgeleitet ist als denen, die in natürlichen Gangliosiden vorhanden sind, und ausgewählt aus der Gruppe bestehend aus einer aliphatischen, einer aromatischen, einer alicyclischen und einer heterocyclischen Gruppe, und die wahlweise durch mindestens eine funktionelle Gruppe substitutiert sein kann.

8. Komplex nach einem der Ansprüche 1-3, worin das Gangliosid ein Gangliosidderivat ist mit einer Acylgruppe, wobei die Acylgruppe von einer anderen Säure abgeleitet ist als denen, die in natürlichen Gangliosiden vorhanden sind, und ausgewählt aus der Gruppe bestehend aus einer aliphatischen, einer aromatischen, einer alicyclischen oder heterocyclischen Gruppe, die wahlweise durch mindestens eine funktionelle Gruppe substitutiert sein kann.

9. Komplex nach einem der Ansprüche 1-3, worin das Gangliosid ein Gangliosidderivat ist mit sowohl einer Sphingosin- und einer Neuraminamidgruppe und worin eine oder beide genannten Gruppen acyliert sind durch eine andere Säure als die, die in natürlichen Gangliosiden vorhanden sind, und worin die Säure ausgewählt ist aus der Gruppe bestehend aus einem aliphatischen Radikal, einem aromatischen Radikal, einem alicyclischen Radikal und einem heterocyclischen Radikal, und die wahlweise durch mindestens eine funktionelle Gruppe substitutiert sein kann.

10. Komplex nach einem der Ansprüche 1-3, worin das halbsynthetische Analoge eines Gangliosids ausgewählt ist aus der Gruppe bestehend aus halbsynthetischen Analogen von GM1, GD1a, GD1b und GT1b.

11. Komplex nach Anspruch 1, worin das Gangliosid ein aliphatischer Ester von Gangliosid GM1 ist, wobei der aliphatische Ester maximal 8 Kohlenstoffatome aufweist.

12. Komplex nach Anspruch 1, worin das Gangliosid ein aliphatisches Amid von GM1 ist, wobei das aliphatische Amid maximal 8 Kohlenstoffatome aufweist.

13. Komplex nach Anspruch 1, worin das Gangliosid ein Peracetylat, ein Perpropionylat, ein Perbutyrat, ein Pervalerianylat, ein Persuccinylat oder ein Permalonylat des Gangliosids GM1 ist.

14. Komplex nach einem der Ansprüche 1-13, der ein βNGF-GM1-Komplex ist.

15. Komplex nach einem der Ansprüche 1-13, der ein βNGF-GD1a-Komplex ist.

16. Komplex nach einem der Ansprüche 1-13, der ein βNGF-GD1b-Komplex ist.

17. Komplex nach einem der Ansprüche 1-13, der ein βNGF-GT1b-Komplex ist.

18. Komplex nach einem der Ansprüche 1-13, der ein Komplex von βNGF und einem inneren Ester von GM1 ist.

19. Verfahren zur Herstellung eines Komplexes nach Anspruch 1, umfassend in Kontakt bringen der β-Untereinheit von NGF mit einem Gangliosid in einer wäßrigen Lösung in Gegenwart oder Abwesenheit anderer organischer Lösungsmittel, bei Raumtemperatur oder etwas darüber, unter alkalischen Bedingungen; und Aufnehmen des Komplexes.

**20.** Verfahren nach Anspruch 19, worin das organische Lösungsmittel ein niederes Dialkylamid oder Diethylsulfoxid ist.

**21.** Komplex zwischen der *β*-Untereinheit des neuronotrophen Faktors NGF und einem natürlichen Gangliosid oder einem halbsynthetischen Analogen eines Gangliosids nach einem der Ansprüche 1-18 in Hinblick auf seine therapeutische Verwendung.

**22.** Komplex nach Anspruch 21 zur Behandlung von neuropathologischen Zuständen.

**23.** Komplex nach Anspruch 21 oder 22 zur Behandlung des Alterns des Nervensystems.

**24.** Komplex nach Anspruch 21 oder 22 zur Behandlung chronischer neurodegenerativer Erkrankung.

**25.** Komplex nach Anspruch 21 oder 22 zur Behandlung chronischer immunologischer Erkrankung, die das Nervensystem betrifft.

**26.** Pharmazeutische Zusammensetzung umfassend eine wirksame Menge eines Komplexes zwischen der *β*-Untereinheit von NGF und einem natürlichen Gangliosid assoziiert durch seine NANA-Gruppe oder einem halbsynthetischen Analogen eines Gangliosids oder einem physiologisch akzeptablen Salz davon; und einen pharmazeutisch akzeptablen Träger.

**27.** Verwendung eines Komplexes zwischen der *β*-Untereinheit des neuronotrophen Wachstumsfaktors NGF und einem Gangliosid nach Anspruch 24 zur Herstellung einer pharmazeutischen Zusammensetzung zur Behandlung von neuropathologischen Zuständen.

**Revendications**

**1.** Un complexe qui consiste en la sous-unité *β* du NGF et en un ganglioside, associé par l'intermédiaire de son groupe NANA, choisi dans le groupe consistant en un ganglioside naturel ou un analogue semisynthétique dudit ganglioside naturel ou un sel physiologiquement acceptable sous forme substantiellement pure en dérivant.

**2.** Le complexe selon la revendication 1, dans lequel le rapport pondéral entre ledit *β*NGF et ledit ganglioside est compris entre 1/100 000 et 1/10.

**3.** Le complexe selon la revendication 2, dans lequel le rapport pondéral entre ledit *β*NGF et ledit ganglioside est d'environ 1/1000.

**4.** Le complexe selon l'une quelconque des revendications 1 à 3, dans lequel ledit ganglioside est un ganglioside naturel choisi dans les groupes consistant en GM1, GD1a, GD1b et GT1b.

**5.** Le complexe selon l'une quelconque des revendications 1 à 3, dans lequel ledit ganglioside est un lysoganglioside.

**6.** Le complexe selon l'une quelconque des revendications 1 à 3, dans lequel ledit ganglioside est un N-acétylganglioside ou un N-acétyl-lysoganglioside.

**7.** Le complexe selon l'une quelconque des revendications 1 à 3, dans lequel ledit ganglioside présente un groupe sphingosine acyle dans lequel ledit groupe acyle est dérivé d'un acide autre que ceux présents dans les gangliosides naturels et choisi dans le groupe consistant en les groupes aliphatique, aromatique, alicyclique et hétérocyclique qui peuvent être éventuellement substitués par au moins un groupe fonctionnel.

**8.** Un complexe selon l'une quelconque des revendications 1 à 3, dans lequel ledit ganglioside est un dérivé de ganglioside ayant un groupe acyle, ledit groupe acyle étant dérivé d'un acide autre que ceux présents dans les gangliosides naturels et choisis dans le groupe consistant en les groupes aliphatique, aromatique, alicyclique et hétérocyclique qui peuvent être éventuellement substitués par au moins un groupe fonctionnel.

EP 0 433 113 B1

**9.** Le complexe selon l'une quelconque des revendications 1 à 3, dans lequel ledit ganglioside est un dérivé d'un ganglioside ayant à la fois un groupe sphingosine et un groupe amide neuraminique et dans lequel l'un ou les deux de ces groupes sont acylés par un acide autre que ceux présents dans les gangliosides naturels et dans lequel ledit acide est choisi dans le groupe consistant en un radical aliphatique, aromatique, alicyclique et hétérocyclique et qui peut être éventuellement substitué par au moins un groupe fonctionnel.

**10.** Le complexe selon l'une quelconque des revendications 1 à 3, dans lequel ledit analogue semi-synthétique du ganglioside est choisi dans le groupe consistant en les analogues semi-synthétiques de GM1, GD1a, GD1b et GT1b.

**11.** Le complexe selon la revendication 1, dans lequel ledit ganglioside est un ester aliphatique du ganglioside GM1, ledit ester aliphatique ayant un maximum de 8 atomes de carbone.

**12.** Le complexe selon la revendication 1, dans lequel ledit ganglioside est un amide aliphatique de GM1, ledit amide ayant un maximum de 8 atomes de carbone.

**13.** Le complexe selon la revendication 1, dans lequel ledit ganglioside est un peracétylate, un perpropionylate, un perbutyrate, un pervalérianylate, un persuccinylate ou un permalonylate du ganglioside GM1.

**14.** Le complexe selon l'une quelconque des revendications 1 à 13, qui est le complexe $\beta$NGF-GM1.

**15.** Le complexe selon l'une quelconque des revendications 1 à 13, qui est le complexe $\beta$NGF-GD1a.

**16.** Le complexe selon l'une quelconque des revendications 1 à 13, qui est le complexe $\beta$NGF-GD1b.

**17.** Le complexe selon l'une quelconque des revendications 1 à 13, qui est le complexe $\beta$NGF-Gt1b.

**18.** Le complexe selon l'une quelconque des revendications 1 à 13, qui est le complexe $\beta$NGF-ester interne de GM1.

**19.** Un procédé pour la préparation d'un complexe selon la revendication 1, qui comprend la mise en contact de la sous-unité $\beta$NGF avec un ganglioside dans une solution aqueuse en présence ou en l'absence d'autres solvants organiques, à température ambiante ou légèrement au-dessus, dans des conditions alcalines; et à collecter ledit complexe.

**20.** Le procédé selon la revendication 19, dans lequel ledit solvant organique est un dialkylamide inférieur ou du diéthylsulfoxyde.

**21.** Le complexe entre la sous-unité $\beta$ du facteur neurotropique NGF et un ganglioside naturel ou un analogue semi-synthétique d'un ganglioside selon l'une quelconque des revendications 1 à 18 pour son utilisation thérapeutique.

**22.** Le complexe selon la revendication 21, pour le traitement de maladies neuropathologiques.

**23.** Le complexe selon la revendication 21 ou 22, pour le traitement du vieillissement du système nerveux.

**24.** Le complexe selon l'une des revendications 21 ou 22, pour le traitement des troubles neurodégénératifs chroniques.

**25.** Le complexe selon la revendication 21 ou 22, pour le traitement des maladies immunologiques chroniques atteignant le système nerveux.

**26.** Une composition pharmaceutique qui comprend une quantité efficace d'un complexe entre la sous-unité $\beta$ du NGF et un ganglioside naturel, associé par son groupe NANA, ou un analogue semi-synthétique d'un ganglioside ou un sel pharmaceutiquement acceptable en dérivant; et un support pharmaceutiquement acceptable.

18

27. Utilisation d'un complexe entre la sous-unité $\beta$ du facteur de croissance neurotropique NGF et d'un ganglioside selon la revendication 24 pour la préparation d'une composition pharmaceutique pour le traitement de maladies neuropathologiques.

Figure 1

Figure 2